(19) Europäisches Patentamt
European Patent Office
Office européen des brevets

(11) **EP 2 962 637 B1**

(12) **EUROPEAN PATENT SPECIFICATION**

(45) Date of publication and mention
of the grant of the patent:
**04.11.2020  Bulletin 2020/45**

(51) Int Cl.:
***A61B 5/11*** *(2006.01)*      ***A61B 5/00*** *(2006.01)*

(21) Application number: **14876668.6**

(22) Date of filing: **25.12.2014**

(86) International application number:
**PCT/CN2014/001178**

(87) International publication number:
**WO 2015/100706 (09.07.2015 Gazette 2015/27)**

(54) **HUMAN MOTION STATUS MONITORING METHOD AND DEVICE**

VERFAHREN UND VORRICHTUNG ZUR ÜBERWACHUNG EINES MENSCHLICHEN BEWEGUNGSSTATUS

PROCÉDÉ ET DISPOSITIF DE SURVEILLANCE DE L'ÉTAT D'UN MOUVEMENT HUMAIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **31.12.2013  CN 201310754081**

(43) Date of publication of application:
**06.01.2016  Bulletin 2016/01**

(73) Proprietor: **Goertek Inc.**
**Hi-Tech Industry District**
**Weifang, Shandong 261031 (CN)**

(72) Inventors:
• **LI, Bo**
**Weifang**
**Shandong 261031 (CN)**
• **WANG, Fupo**
**Weifang**
**Shandong 261031 (CN)**
• **LI, Na**
**Weifang**
**Shandong 261031 (CN)**

(74) Representative: **LKGLOBAL**
**Lorenz & Kopf PartG mbB Patentanwälte**
**Brienner Straße 11**
**80333 München (DE)**

(56) References cited:
**EP-A1- 2 236 081        EP-A2- 0 700 661**
**CN-A- 1 723 848        CN-A- 101 394 787**
**CN-A- 103 767 710        US-A1- 2009 240 155**
**US-A1- 2013 173 174        US-A1- 2013 173 174**

## Description

## Field of the Invention

The present invention relates to the field of sports equipment, in particular, relates to a human body movement state monitoring method and device.

## Background of the Invention

With the continuous development of social economy, material living standard is gradually improved, and at the same time, health of ones own is more and more concerned , and a variety of sports schemes for fitness are customized for ones own. Therefore, various devices for monitoring sports schemes occur.

Pedometer is a device capable of calculating the walk or run step number of its wearer. However, pedometer merely has a rather single function, which cannot monitor other movement forms and sleeping of a person. There are also some devices capable of monitoring various forms of movement of a person comprehensively, which require the intervention of human operation, and fail to monitor various movement conditions and sleeping conditions of the person automatically, comprehensively, and round-the-clock. Meanwhile, sleeping conditions are very helpful for determining the health conditions of the person. During sleeping, the person may make some abnormal movements such as turning, scratching, scaring, etc.. If such abnormal movements occur rather frequently, it indicates that the person's sleep quality is not good enough. Therefore, both the monitoring of person's sleeping conditions and normal movement conditions are helpful for improving one's fitness schemes.

The document EP 0 700 661 A2 relates to an exercise amount measuring device comprising an acceleration sensor for detecting a body movement of a living body, means for calculating an exercise amount based on a detection signal of the acceleration sensor, and a display section for displaying the calculated exercise amount, wherein a remaining target calorie value or a target exercise amount is calculated and displayed.

## Summary of the Invention

The present invention is aimed to solve the problems existing in the aforesaid prior art, with an object for providing a human body movement state monitoring method and device, the method and device can automatically, comprehensively, round-the-clock, accurately monitor various movement states of a person, thus provide basis for improving one's fitness schemes.

For achieving aforesaid object, one aspect of the present invention provides a human body movement state monitoring method, the method comprising the following steps performed repeatedly:

a) obtaining acceleration signals having a set sampling time period from output of a triaxial acceleration sensor worn on a human body, and calculating the energy and average power of the acceleration signals;

b) determining a human body movement state according to the average power of the acceleration signals, and if the average power of the acceleration signals is more than a predetermined fierce movement threshold, determining that the human body is in a fierce movement state, if the average power of the acceleration signals is less than a predetermined sleeping threshold, determining that the human body is in a sleeping state, if the average power of the acceleration signals is less than the fierce movement threshold and is more than the sleeping threshold, determining that the human body is in a light movement state;

c1) if the human body is in the sleeping state, accumulating the time periods of the acceleration signals into the total time period of the sleeping state, accumulating the energy of the acceleration signals into the total energy of the sleeping state, counting up the time periods of acceleration signals which have intensity more than a predetermined intensity threshold, and accumulating the counted time periods into the total time period of sleeping abnormal movements, and setting the sampling time period of acceleration signals as the sampling time period of the sleeping state, then returning to step a);

c2) if the human body is in the light movement state, accumulating the time periods of the acceleration signals into the total time period of the light movement state, accumulating the energy of the acceleration signals into the total energy of the light movement state, and setting the sampling time period of acceleration signals as the sampling time period of the light movement state, then returning to step a);

c3) if the human body is in the fierce movement state, further determining whether the acceleration signals have quasi-periodicity, if the acceleration signals do not have quasi-periodicity, determining that the human body is in an irregular fierce movement state, if the acceleration signals have quasi-periodicity, determining that the human body is in a regular fierce movement state;

d1) if the human body is in the irregular fierce movement state, accumulating the time periods of the acceleration signals into the total time period of the irregular fierce movement state, accumulating the energy of the acceleration signals into the total energy of the irregular fierce movement state, and setting the sampling time period of acceleration signals as the sampling time period of the fierce movement state, then returning to step a);

d2) if the human body is in the regular fierce movement state, accumulating the time periods of the acceleration signals into the total time period of the regular fierce movement state, accumulating the energy of the acceleration signals into the total energy of the regular fierce movement state, calculating movement step number according to the acceleration signals, and accumulating the movement step number into the total movement step number, and setting the sampling time period of acceleration signals as the sampling time period of the fierce movement state, then returning to step a).

The average power P of the acceleration signals is calculated from the following formula:

$$P = \frac{1}{N} \sum_{i=1}^{N} \left( a_i - a_0 \right)^2$$

wherein $a_i$ is the No. i value of the acceleration signals, N is the length of the acceleration signals, and $1 \leq i \leq N$, $a_0$ is the average value of the acceleration signals,

$$a_0 = \frac{1}{N} \sum_{i=1}^{N} a_i.$$

Preferably, the determining step of the quasi-periodicity of the acceleration signals can comprise:

① performing high-pass filtering on the acceleration signals;

② performing pitch detection on the high-pass filtered acceleration signals;

③ setting a low-pass or band-pass filter by using the pitch obtained by the pitch detection as cut-off frequency, and using the low-pass or band-pass filter to perform low-pass or band-pass filtering on corresponding high-pass filtered acceleration signals;

④ obtaining extreme value points of the acceleration signals in the low-pass or band-pass filtered acceleration signals and removing interfering extreme value points in the extreme value points of the acceleration signals, so as to obtain effective extreme value points in the low-pass or band-pass filtered acceleration signals;

⑤ calculating time gaps between adjacent effective extreme value points, obtaining a time gap sequence, and calculating differences between adjacent time gaps in the time gap sequence, obtaining a time gap difference sequence, and if each of a continuous predetermined number of time gap differences in the time gap difference sequence is less than a predetermined period threshold, determining that the acceleration signals have quasi-periodicity, otherwise, determining that the acceleration signals do not have quasi-periodicity.

Further preferably, the step of calculating movement step number according to the acceleration signals can comprise:

counting the effective extreme value points in the low-pass or band-pass filtered acceleration signals having quasi-periodicity, the number of the effective extreme value points being movement step number.

Further preferably, a displacement signal can be obtained by double integral of the acceleration signals on time.

The method of performing pitch detection on the high-pass filtered acceleration signals can comprise one or more of autocorrelation function method, cepstrum method, linear predictive coding method and average magnitude difference function method.

Preferably, performing pitch detection on the high-pass filtered acceleration signals can comprise: attenuating the signals with a filter that attenuates signals with an incrementing degree from low frequency to high frequency; obtaining the autocorrelation function $\rho(\tau)$ of the attenuated signals from the following formula:

$$\rho(\tau) = \frac{\sum_{n=1}^{N} a(n) a(n - \tau)}{\sqrt{\sum_{n=1}^{N} a^2(n) \sum_{n=0}^{N-1} a^2(n - \tau)}}$$

wherein $a(n)$ is the No. n value of the attenuated signals, N is the length of the signals, and $1 \leq n \leq N$, $\tau$ is a delay time, $\rho(\tau)$ is normalized autocorrelation function of the signals; calculating the value of $\tau$ corresponding to the maximal value of $\rho(\tau)$, and the reciprocal of the $\tau$ value is the pitch of the signals.

Wherein, removing the interfering extreme value points from the extreme value points of the acceleration signals comprises: filtering out the interfering extreme value points from the extreme value points of the acceleration signals through a time gap; alternatively, filtering out the interfering extreme value points from the extreme value points of the acceleration signals through a time gap and a magnitude value.

Preferably, the interfering extreme value points may comprise such an extreme value point of the acceleration signals that the time gap between the extreme value point of the acceleration signals and the previous extreme value point of the acceleration signals is less than a predetermined threshold; or the interfering extreme value points may comprise extreme value points of the acceleration signals, whose magnitude values are not maximal, among each group of extreme value points of the acceleration signals with time gaps continuously less than a predetermined threshold.

Preferably, the human body movement state monitoring method also can comprise optionally displaying the total time period of the sleeping state, the total energy of the sleeping state, the total time period of the sleeping abnormal movements, the total time period of the light movement state, the total energy of the light movement state, the total time period of the irregular fierce movement state, the total energy of the irregular fierce movement state, the total time period of the regular fierce movement state, the total energy of the regular fierce movement state and the total step number of movement.

According to another aspect of the present invention, a human body movement state monitoring device is provided, which comprises: a triaxial acceleration sensor, an acceleration signal obtaining unit, a calculating unit, a human body movement state determining unit, a sleeping abnormal movement statistical unit, a sampling time period setting unit, a storage unit, a quasi-periodicity determining unit, and a step counting unit.

The acceleration signal obtaining unit obtains acceleration signals having a set sampling time period from output of the triaxial acceleration sensor worn on a human body, and the calculating unit calculates the energy and average power of the acceleration signals;

the human body movement state determining unit determines a human body movement state according to the average power of the acceleration signals, and if the average power of the acceleration signals is more than a predetermined fierce movement threshold, determines that the human body is in a fierce movement state, if the average power of the acceleration signals is less than a predetermined sleeping threshold, determines that the human body is in a sleeping state, if the average power of the acceleration signals is less than the fierce movement threshold and is more than the sleeping threshold, determines that the human body is in a light movement state;

if the human body movement state determining unit determines that the human body is in the sleeping state, accumulates the time periods of the acceleration signals into the total time period of the sleeping state, accumulates the energy of the acceleration signals into the total energy of the sleeping state, the sleeping abnormal movement statistical unit counts up the time periods of acceleration signals which have intensity more than a predetermined intensity threshold, and accumulates the counted time periods into the total time period of sleeping abnormal movements, the sampling time period setting unit sets the sampling time period of acceleration signal as the sampling time period of the sleeping state, the storage unit stores the total time period of the sleeping state, the total energy of the sleeping state and the total time period of the sleeping abnormal movements;

if the human body movement state determining unit determines that the human body is in the light movement state, accumulates the time periods of the acceleration signals into the total time period of the light movement state, accumulates the energy of the acceleration signals into the total energy of the light movement state, and the sampling time period setting unit sets the sampling time period of acceleration signal as the sampling time period of the light movement state, the storage unit stores the total time period of the light movement state and the total energy of light movement state;

if the human body movement state determining unit determines that the human body is in the fierce movement state, then the quasi-period determining unit determines whether the acceleration signals have quasi-periodicity, and if determining that the acceleration signals do not have quasi-periodicity, then the human body movement state determining unit determines that the human body is in an irregular fierce movement state, and if the quasi-period determining unit determines that the acceleration signals have quasi-periodicity, then the human body movement state determining unit determines that the human body is in a regular fierce movement state;

if the human body movement state determining unit determines that the human body is in the irregular fierce movement state, then accumulates the time periods of the acceleration signals into the total time period of the irregular fierce movement state, accumulates the energy of the acceleration signals into the total energy of the irregular fierce movement state, and the sampling time period setting unit sets the sampling time period of acceleration signals as the sampling time period of the fierce movement state, the storage unit stores the total time period of the irregular fierce movement state and the total energy of the irregular fierce movement state;

if the human body movement state determining unit determines that the human body is in the regular fierce movement state, then accumulates the time periods of the acceleration signals into the total time period of the regular fierce movement state, accumulates the energy of the acceleration signals into the total energy of the regular fierce movement state, and the step counting unit calculates movement step number according to the acceleration signals, and accumulates the movement step number into the total movement step number, the sampling time period setting unit sets the sampling time period of acceleration signal as the sampling time period of fierce movement state, the storage unit stores the total time period of the regular fierce movement state, the total energy of the regular fierce movement state and the movement step number.

Preferably, the human body movement state monitoring device may further comprise a display unit for optionally displaying the total time period of the sleeping state, the total energy of the sleeping state, the total time period of the sleeping abnormal movements, the total time period of the light movement state, the total energy of the light movement state, the total time period of the irregular fierce movement state, the total energy of the irregular fierce movement state, the total time period of the regular fierce movement state, the total energy of the regular fierce movement state and the total step number of movement.

It is known from the above description and practice that the human body movement state monitoring method and device of the present invention can automatically, comprehensively, round-the-clock, accurately monitor various movement states (including sleeping state) of a person, can measure the quality of human sleep quantitatively, measure their movement step number during walking and running accurately, and can measure their movement level and energy consumption conditions quantitatively.

The aforesaid description is merely the summary of technical solution of the present invention, for understanding the technical means of the present invention more clearly, it can be carried out according to the disclosure of specification, and for making aforesaid and other objectives, features and advantages of the present invention more apparent to be understood, embodiments of the present invention are listed as follows.

**Brief Description of Drawings**

By reading the detailed description of the preferable embodiment hereinafter, all the other features and advantages will be apparent to the general skilled in the art. The attached drawings are merely for the purpose of demonstrating preferable embodiment, rather than deemed as a limitation of the present invention. Among the attached drawings:

Fig. 1 is a signal graph, demonstrating the example of acceleration signals produced by a triaxial acceleration sensor in three directions during sleeping of its wearer;

Fig.2 is a signal graph, demonstrating the example of acceleration signals produced by the triaxial acceleration sensor in three directions during light movement of its wearer;

Fig.3 is a signal graph, demonstrating the example of acceleration signals produced by the triaxial acceleration sensor in three directions during irregular fierce movement of its wearer;

Fig.4 is a signal graph, demonstrating the example of acceleration signals produced by the triaxial acceleration sensor in three directions during regular fierce movement of its wearer;

Fig.5 is a block diagram, demonstrating a human body movement state monitoring method according to one embodiment;

Fig.6a is a signal graph, demonstrating representative normalized acceleration signals having a predetermined length output from the triaxial acceleration sensor;

Fig.6b is a signal graph, demonstrating high-pass filtered acceleration signals;

Fig.6c is a signal graph, demonstrating low-pass filtered acceleration signals;

Fig.6d is a signal graph, demonstrating an example of extreme value points of the low-pass filtered acceleration signals;

Fig.7 demonstrating an example of frequency response curve of a filter that attenuates signals with an incrementing degree from low frequency to high frequency;

Fig.8 is a signal graph, demonstrating another example of extreme value points of low-pass filtered monoaxial acceleration signals;

Fig.9 is a block diagram, demonstrating a human body movement state monitoring device according to one embodiment;

Fig.10 illustratively demonstrates a block diagram of a server for carrying out the method according to the present invention; and

Fig.11 illustratively demonstrates a storage unit for maintaining or carrying the program codes for achieving the method according to the present invention.

**Detailed Description of the Invention**

The present invention will be described in details in combination with the attached drawings and specific examples.

In the following description, certain illustrative examples of the present invention are only described by way of illustration. Undoubtedly, one of ordinary skilled in the art may recognize that the examples can be amended by using a variety of different ways without departing from the spirit and scope of the present invention. Accordingly, the attached drawings and descriptions are illustrative in nature, and not intended to limit the protection scope of the claims. In the present specification, the same reference numerals denote the same or similar parts.

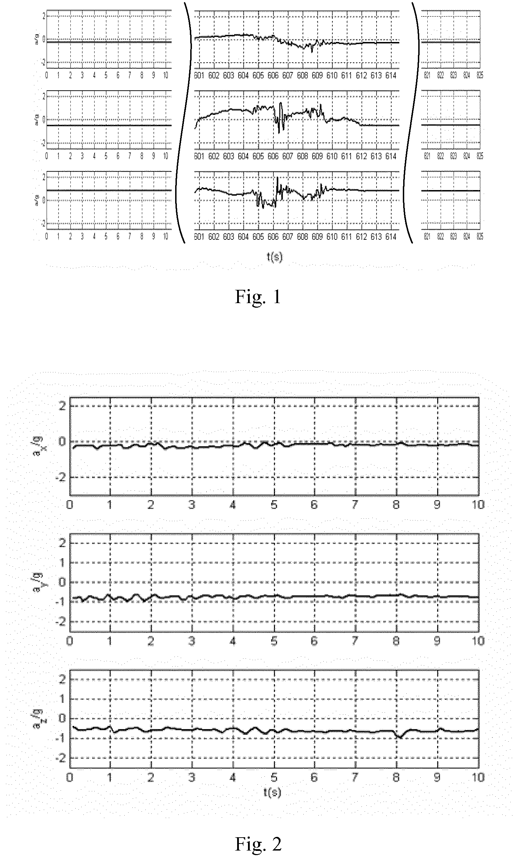

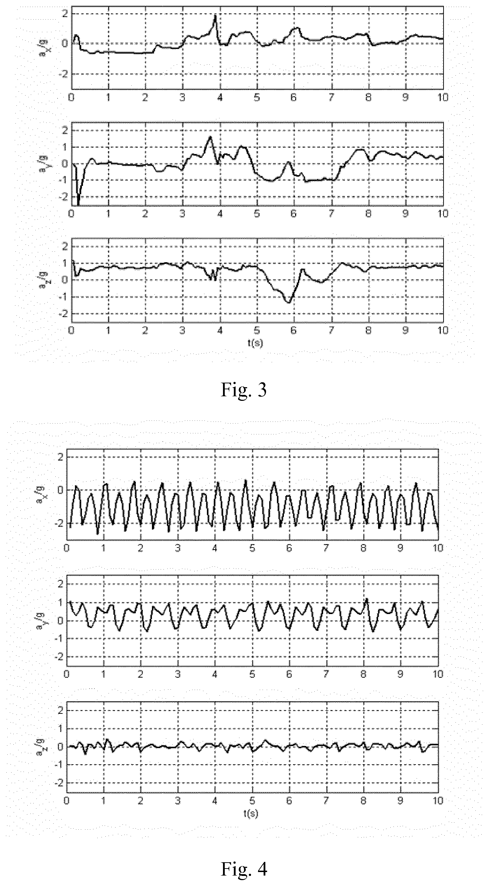

The human body movement state monitoring method of the present invention is carried out by using a device having a triaxial acceleration sensor. Figs. 1-4 are signal graphs, respectively demonstrating the example of acceleration signals produced by the triaxial acceleration sensor in three directions during sleeping (including light sleep, deep sleep), light movements (such as typewriting, unconscious human body shaking, etc.), irregular fierce movements (manual labor, playing basketball, etc.) and regular fierce movements (walking, running, jumping rope, gym body building, etc.) of its wearer. As shown in Figs. 1-4, generally speaking, the orientation of the device comprising the triaxial acceleration sensor in use is not unchanged, thus, during sleeping, light movements and irregular fierce movements, the triaxial output signals of the triaxial acceleration sensor are almost similar, while during regular fierce movements, signal intensity in a certain direction is stronger. In any case, the acceleration signal with the highest energy among the triaxial output of the triaxial acceleration sensor can be selected to measure movement conditions accurately and representatively. Therefore, in the description of the present invention, the acceleration signals output from the triaxial acceleration sensor can denote the acceleration signal with the highest energy among the triaxial output, but the present invention is not limited to this, and also may denote acceleration signals after triaxial output is fused in either way. Alternatively, it can also be, after obtaining corresponding measurement amount from each monoaxial acceleration signal of triaxial acceleration sensor, performing weighting and averaging in a certain weighting way, and finally obtaining a total measurement amount.

As shown in Figs. 1-3, during sleeping, light movements and irregular fierce movements, the common feature of the acceleration signals output from the triaxial acceleration sensor is not having quasi-periodicity, therefore, it is possible to quantitatively measure the movement amount of these movement manners by measuring the duration and total energy of these signals. During the regular fierce movements, the feature of the acceleration signals output from the triaxial acceleration sensor is having quasi-periodicity, therefore, in addition to capable of measuring its duration and total energy, it is also able to further measure its period number, the period number corresponding to the step number of running, the number of jumping, the number of push-pull etc., and in the present invention, these amounts are referred to as movement step number. In another aspect, during sleeping, light movements and irregular (regular) fierce movements, different feature of the acceleration signals output from the triaxial acceleration sensor is different fierce degree of the signal change, which not only presented in the intensity scale of the acceleration signals, but also presented in the time scale. For example, during sleeping, the acceleration signals during most time are very small and gentle, the time with abnormal movements occurring during sleeping (For example, in Fig.1 the turning-over movements occurring during the time period of 600-610 seconds) accounts for a very small ratio of whole sleeping time. Therefore, when analyzing the acceleration signals produced during sleeping, sampling length of signal should be relatively long, which not only facilitates for reducing computation amount, improving analyzing speed, but also makes abnormal movements during sleeping separable from the movements during light movements and fierce movements, this is because, since the time of abnormal movements accounts for a relatively small ratio of whole sleeping time, the contribution of abnormal movements of sleeping to the average power is negligible. Similarly, when analyzing the acceleration signals produced during light movements and fierce movements, sampling length should be different, for reducing computation amount, improving analyzing speed and also not enabling corresponding acceleration signals to lose feature.

One embodiment of the present invention provides a human body movement state monitoring method. The method comprises the following steps performed repeatedly:

step a) obtaining acceleration signals having a set sampling time period from output of a triaxial acceleration sensor worn on a human body, and calculating the energy and average power of the acceleration signals, turning to step b);

step b) determining a human body movement state according to the average power of the acceleration signals, and if the average power of the acceleration signals is more than a predetermined fierce movement threshold, it is determined that the human body is in a fierce movement state, turning to step c3), if the average power of the acceleration signals is less than a predetermined sleeping threshold, it is determined that the human body is in a sleeping state, turning to step c1); if the average power of the acceleration signals is less than the fierce movement threshold and is more than the sleeping threshold, it is determined that the human body is in a light movement state, turning to step c2);

step c1) if the human body is in the sleeping state, accumulating the time periods of the acceleration signals into the total time period of the sleeping state, accumulating the energy of the acceleration signals into the total energy of the sleeping state, counting up the time periods of acceleration signals which have intensity more than a predetermined intensity threshold, and accumulating the counted time periods into the total time period of sleeping abnormal movements, and setting the sampling time period of acceleration signals as the sampling time period of the sleeping state, then returning to step a);

step c2) if the human body is in the light movement state, accumulating the time periods of the acceleration signals into the total time period of the light movement state, accumulating the energy of the acceleration signals into the total energy of the light movement state, and setting the sampling time period of acceleration signals as the sampling time period of the light movement state, then returning to step a);

step c3) if the human body is in the fierce movement state, further determining whether the acceleration signals have quasi-periodicity, if the acceleration signals do not have quasi-periodicity, it is determined that the human body is in an irregular fierce movement state, turning to step d1); if the acceleration signals have quasi-periodicity, it is determined that the human body is in a regular fierce movement state, turning to step d2);

step d1) if the human body is in the irregular fierce movement state, accumulating the time periods of the acceleration signals into the total time period of the irregular fierce movement state, accumulating the energy of the acceleration signals into the total energy of the irregular fierce movement state, and setting the sampling time period of acceleration signal as the sampling time period of the fierce movement state, then returning to step a);

step d2) if the human body is in the regular fierce movement state, accumulating the time periods of the acceleration signals into the total time period of the regular fierce movement state, accumulating the energy of the acceleration signals into the total energy of the regular fierce movement state, calculating movement step number according to the acceleration signals, and accumulating the movement step number into the total movement step number, and setting the sampling time period of acceleration signals as the sampling time period of the fierce movement state, then returning to step a).

The reference sign added to each embodiment of the present invention is merely to facilitate demonstrating the operation order of a preferable solution, but does not limit specific operation sequence of each step strictly.

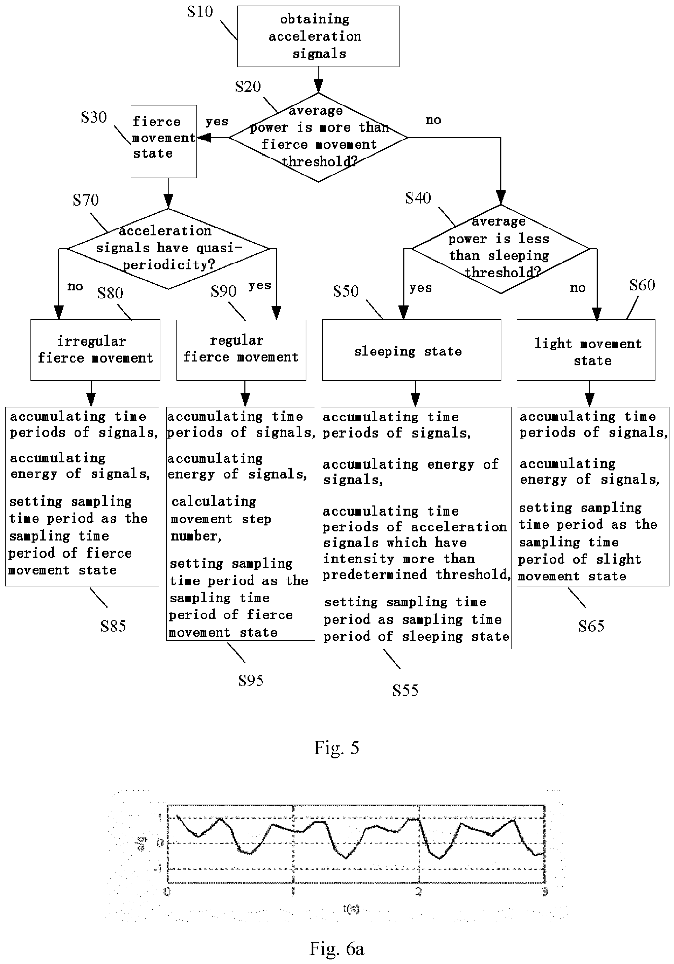

Fig.5 is a block diagram, demonstrating a human body movement state monitoring method according to one embodiment. As shown in Fig.5, the human body movement state monitoring method according to the embodiment comprises the following steps performed repeatedly:

firstly, in step S10, obtaining acceleration signals having a set sampling time period from output of a triaxial acceleration sensor worn on a human body, and calculating the energy and average power of the acceleration signals.

The average power P of the acceleration signals is calculated from the following formula:

$$P = \frac{1}{N} \sum_{i=1}^{N} \left( a_i - a_0 \right)^2$$

wherein $a_i$ is the No. i value of the acceleration signals, N is the length of the acceleration signals, and $1 \leq i \leq N$, $a_0$ is the average value of the acceleration signals,

$$a_0 = \frac{1}{N} \sum_{i=1}^{N} a_i.$$

subsequently, determining a human body movement state according to the average power of the acceleration signals. For example, in step S20, determining whether the average power of the acceleration signals is more than a predetermined fierce movement threshold, and if the average power of the acceleration signals is more than the predetermined fierce movement threshold, it is determined that the human body is in a fierce movement state (step S30), otherwise, in step S40, determining whether the average power of the acceleration signals is less than a predetermined sleeping threshold, and if the average power of the acceleration signals is less than the predetermined sleeping threshold, it is determined that the human body is in a sleeping state (step S50), if the average power of the acceleration signals is less than the fierce movement threshold and is more than the sleeping threshold, it is determined that the human body is in a light movement state (step S60). Wherein, the fierce movement threshold and the sleeping threshold can be obtained according to experiments, and can be adjusted.

If the human body is in the sleeping state, then in step S55, accumulating the time periods of the acceleration signals into the total time period of the sleeping state, accumulating the energy of the acceleration signals into the total energy of the sleeping state, counting up the time periods of acceleration signals which have intensity more than a predetermined intensity threshold, and accumulating the counted time periods into the total time period of sleeping abnormal movements, and setting the sampling time period of acceleration signals as the sampling time period of the sleeping state, then returning to step S10. It should be noted that, the time period of acceleration signals which has intensity more than a predetermined intensity threshold denotes such a period of time that within this period of time, the intensity size of the acceleration signals is more than the predetermined intensity threshold. This period of time is the time of abnormal movements of sleeping, during which abnormal movements of sleeping such as turning-over, scaring, spasm etc. occur, and through analyzing the ratio of total time period of abnormal movement time of sleeping accounting for total time period of sleeping, sleeping quality can be analyzed quantitatively, and when the ratio is very small, it denotes that the sleeping is deep sleeping, when the ratio is relatively large, it denotes that the sleeping is light sleeping. In addition, the sampling time period of sleeping state can be determined according to experiments, such as 5-10 minutes.

If the human body is in the light movement state, then in step S65, accumulating the time periods of the acceleration signals into the total time period of the light movement state, accumulating the energy of the acceleration signals into the total energy of the light movement state, and setting the sampling time period of acceleration signals as the sampling time period of the light movement state, then returning to step S10. The sampling time period of light movement state can be determined according to experiment, such as 1 minute.

If the human body is in the fierce movement state, then in step S70 further determining whether the acceleration signals have quasi-periodicity, if the acceleration signals do not have quasi-periodicity, it is determined that the human body is in an irregular fierce movement state (step S80), if the acceleration signals have quasi-periodicity, it is determined that the human body is in a regular fierce movement state (step 90).

Wherein, the determination of the quasi-periodicity of the acceleration signals can comprise the following steps CD to ⑤:

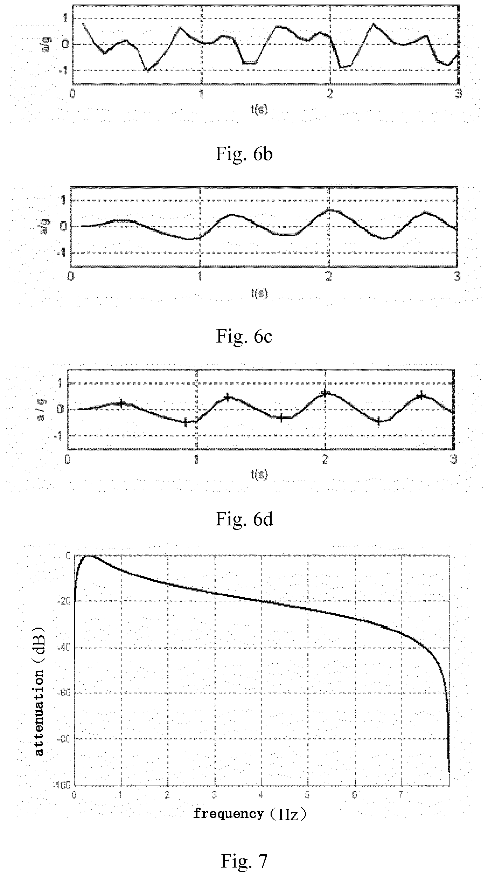

Step ① performing high-pass filtering on the acceleration signals. Since the acceleration signals output from the triaxial acceleration sensor generally comprise DC component, and the existence of the DC component would interfere with the analyzing of acceleration signals, the DC component is removed from the acceleration signals through high-pass filtering. Fig.6a is a signal graph, demonstrating the representative normalized acceleration signal a/g having a predetermined length output from the triaxial acceleration sensor, wherein, a denotes acceleration, g denotes gravity acceleration. Fig.6b is a signal graph, demonstrating high-pass filtered acceleration signals. It can be seen from Fig.6b that after high-pass filtering, the acceleration signals only comprise AC component.

Step ② performing pitch detection on the high-pass filtered acceleration signals. The acceleration signals may comprise various frequency components corresponding to different body rhythmic movements, such as pitch component, frequency multiplication component and other high frequency components. Fig.7 is schematic diagram of spectrum of acceleration signals. Wherein, pitch component is related to most fundamental rhythmic movements, and determining quasi-periodicity of signals according to the pitch component would be more accurate. For obtaining the acceleration signals only consisting of the pitch component, high frequency component in the acceleration signals is required to be filtered out. And in order to filter high frequency component, frequency of pitch component is required to be measured roughly, so as to configure a suitable filter for filtering high frequency component outside of the pitch component.

There are various methods for pitch detection, e.g., conventional methods in voice signal pitch detection such as autocorrelation function method, cepstrum method, linear predictive coding method, average magnitude difference function method can be used. Preferably, autocorrelation function method can be used.

Specifically, the high-pass filtered acceleration signals are attenuated with a filter that attenuates signal energy with an incrementing degree from low frequency to high frequency, to suppress high frequency component in the acceleration signals, so as to highlight the pitch component in the monoaxial acceleration signals, and reduce the error of obtained pitch. Fig.7 shows an example of frequency response curve of a filter that attenuates signal energy with an incrementing degree from low frequency to high frequency. After acceleration signals are attenuated with the filter, low-frequency component in the signals is attenuated relatively slightly, while high frequency component is attenuated relatively largely. Thus, when further using autocorrelation function method to obtain pitch of the monoaxial acceleration signals filtered as such, the obtained pitch is relatively accurate.

Then, the autocorrelation function $\rho(\tau)$ of the attenuated signals is obtained from the following formula:

$$\rho(\tau) = \frac{\sum\limits_{n=1}^{N} a(n)a(n-\tau)}{\sqrt{\sum\limits_{n=1}^{N} a^2(n)\sum\limits_{n=1}^{N} a^2(n-\tau)}}$$

wherein $a(n)$ is the No. n value of the attenuated signals, N is the length of the signals, and $1\leq n\leq N$, $\tau$ is a delay time, $\rho(\tau)$ is normalized autocorrelation function of the signals; calculate the value of $\tau$ corresponding to the maximal value of $\rho(\tau)$, and the reciprocal of the $\tau$ value is the pitch of the signals.

Step ③ setting a low-pass or band-pass filter by using the pitch obtained by the pitch detection as cut-off frequency, and using the low-pass or band-pass filter to perform low-pass or band-pass filtering on corresponding high-pass filtered acceleration signals. After low-pass or band-pass filtering, relatively smooth signals can be obtained, to facilitate accurately calculating extreme value points of the acceleration signals. Fig.6c is a signal graph, demonstrating low-pass filtered acceleration signals.

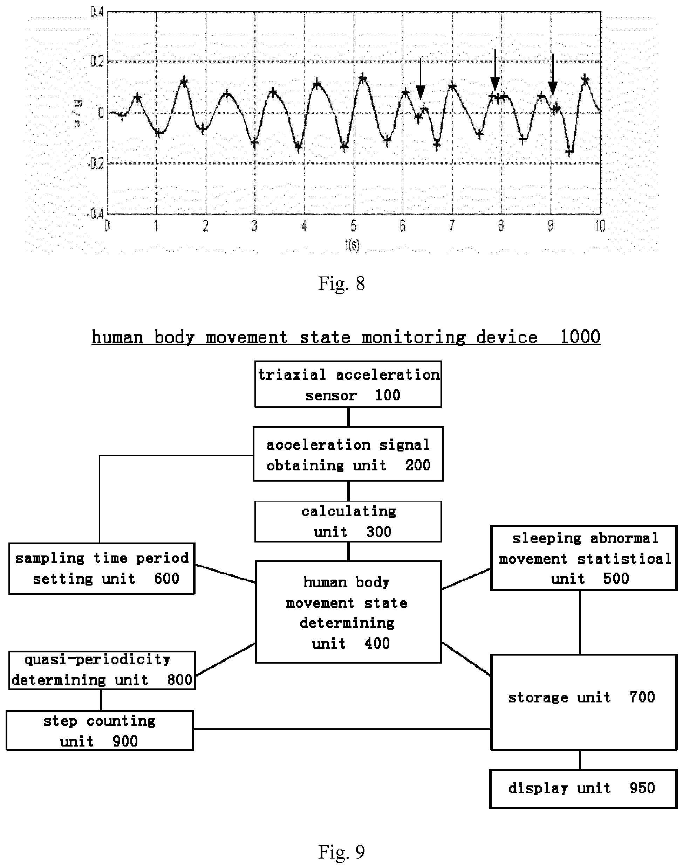

Step ④ obtaining extreme value points of the acceleration signals in the low-pass or band-pass filtered acceleration signals and removing interfering extreme value points in the extreme value points of the acceleration signals, so as to obtain effective extreme value points. Fig.6d is a signal graph, demonstrating an example of extreme value points of low-pass or band-pass filtered monoaxial acceleration signals, wherein, symbol + denotes extreme value point (comprising maximal and minimal value points). Fig.6d demonstrates a rather specific example, wherein, noise interference is almost not existed in the low-pass or band-pass filtered acceleration signals. In a more general case, after low-pass or band-pass filtering, there still exists noise interference in the acceleration signals, which is represented by the existence of interfering extreme value points. Fig.8 is a signal graph, demonstrating another example of extreme value points of low-pass or band-pass filtered acceleration signals. As shown in Fig.8, interfering extreme value points (as indicated by arrow in Fig.8) exists in the low-pass or band-pass filtered monoaxial acceleration signals, and these interfering extreme value points do not represent the extreme value points related to periodic movements, which only result in over-counting of step number, removing these interfering extreme value points will make the counted step number more accurate. Thus, it required to remove these interfering extreme value points, so as to obtain the extreme value point corresponding to walking and running step number accurately.

In one embodiment of the present invention, the interfering extreme value points may comprise such an extreme value point of the acceleration signals that the time gap between the extreme value point of the acceleration signals and the previous extreme value point of the acceleration signals is less than a predetermined threshold, wherein, the predetermined threshold is far less than the period of the pitch component of monoaxial acceleration signals. In the embodiment, in each group of extreme value points relatively close to each other, only one extreme value point on the leftmost is kept, and the other extreme value points are removed as interfering extreme value points. In this manner, the interfering extreme value points in the extreme value points of the acceleration signals are filtered out through the time gap between the extreme value points of the acceleration signals.

In another embodiment of the present invention, the interfering extreme value points may comprise such extreme value points of the acceleration signals, whose magnitude values are not maximal, among each group of extreme value points of the acceleration signals with time gaps continuously less than a predetermined threshold. In another words, in the embodiment, in each group of extreme value points relatively close to each other, only the extreme value point of the acceleration signals with maximal magnitude value is kept, and the other extreme value points are removed as interfering extreme value points. In this manner, the interfering extreme value points in the extreme value points of the acceleration signals are filtered out through the time gaps between the extreme value points of the acceleration signals and the magnitude values of extreme value points of the acceleration signals.

Step ⑤ calculating time gaps between adjacent effective extreme value points, obtaining a time gap sequence, and calculating differences between adjacent time gaps in the time gap sequence, obtaining a time gap difference sequence, and if each of a continuous predetermined number of time gap differences in the time gap difference sequence is less than a predetermined period threshold, it is determined that the acceleration signals have quasi-periodicity, otherwise, it is determined that the acceleration signals do not have quasi-periodicity.

Returning back to Fig.5, if the human body is in the irregular fierce movement state, then in step 85 accumulating the time periods of the acceleration signals into the total time period of the irregular fierce movement state, accumulating the energy of the acceleration signals into the total energy of the irregular fierce movement state, and setting the sampling time period of acceleration signals as the sampling time period of the fierce movement state, then returning to step S10. The sampling time period of fierce movement state may be 1 ~3 seconds.

If the human body is in the regular fierce movement state, then in step S95 accumulating the time periods of the acceleration signals into the total time period of the regular fierce movement state, accumulating the energy of the acceleration signals into the total energy of the regular fierce movement state, calculating movement step number according to the acceleration signals, and accumulating the movement step number into the total movement step number, and setting the sampling time period of acceleration signals as the sampling time period of the fierce movement state, then returning to step S10.

The method of calculating movement step number according to the acceleration signals can comprise: count the effective extreme value points in the low-pass or band-pass filtered acceleration signals having quasi-periodicity, and the number of the effective extreme value points is the movement step number obtained in this step counting process. Furthermore, a displacement signal can be obtained by double integral of the acceleration signals on time, so as to provide reference for actual movement distance to a walker & runner. In addition, it can be distinguished whether it is in situ movements or actual walking & running according to the size of the displacement.

The human body movement state monitoring method of the present invention further comprises optionally displaying the total time period of the sleeping state, the total energy of the sleeping state, the total time period of the sleeping abnormal movements, the total time period of the light movement state, the total energy of the light movement state, the total time period of the irregular fierce movement state, the total energy of the irregular fierce movement state, the total time period of the regular fierce movement state, the total energy of the regular fierce movement state and the total step number of movement. Thus, according to these data, a person's sleeping quality, movement level and energy consumption condition can be known.

Hereinbefore the human body movement state monitoring method of the present invention is described by referring to Figs. 1-8. The human body movement state monitoring method of the present invention can be achieved through software, and also can be achieved through hardware, or achieved in a combination way of software and hardware.

Fig.9 is a block diagram, demonstrating a human body movement state monitoring device according to one embodiment. As shown in Fig.9, a human body movement state monitoring device 1000 according to one embodiment of the present invention comprises: a triaxial acceleration sensor 100, an acceleration signal obtaining unit 200, a calculating unit 300, a human body movement state determining unit 400, a sleeping abnormal movement statistical unit 500, a sampling time period setting unit 600, a storage unit 700, a quasi-periodicity determining unit 800 and a step counting unit 900.

The acceleration signal obtaining unit 200 obtains acceleration signals having a set sampling time period from output of the triaxial acceleration sensor 100 worn on a human body, and the calculating unit 300 calculates the energy and average power of the acceleration signals.

The human body movement state determining unit 400 determines a human body movement state according to the average power of the acceleration signals, and if the average power of the acceleration signals is more than a predetermined fierce movement threshold, determines that the human body is in a fierce movement state; if the average power of the acceleration signals is less than a predetermined sleeping threshold, determines that the human body is in a sleeping state; if the average power of the acceleration signals is less than the fierce movement threshold and is more than the sleeping threshold, determines that the human body is in a light movement state;

If the human body movement state determining unit 400 determines that the human body is in the sleeping state, accumulates the time periods of the acceleration signals into the total time period of the sleeping state, accumulates the energy of the acceleration signals into the total energy of the sleeping state; the sleeping abnormal movement statistical unit 500 counts up the time periods of acceleration signals which have intensity more than a predetermined intensity threshold, and accumulates the counted time periods into the total time period of sleeping abnormal movements; the sampling time period setting unit 600 sets the sampling time period of acceleration signals as the sampling time period of the sleeping state; the storage unit 700 stores the total time period of the sleeping state, the total energy of the sleeping state and the total time period of the sleeping abnormal movements.

If the human body movement state determining unit 400 determines that the human body is in the light movement state, accumulates the time periods of the acceleration signals into the total time period of the light movement state, accumulates the energy of the acceleration signals into the total energy of the light movement state, and the sampling time period setting unit 600 sets the sampling time period of acceleration signals as the sampling time period of the light movement state; the storage unit 700 stores the total time period of the light movement state and the total energy of light movement state.

If the human body movement state determining unit 400 determines that the human body is in the fierce movement state, then the quasi-period determining unit 800 determines whether the acceleration signals have quasi-periodicity, and if determining that the acceleration signals do not have quasi-periodicity, then the human body movement state determining unit 400 determines that the human body is in an irregular fierce movement state, and if the quasi-period determining unit 800 determines that the acceleration signals have quasi-periodicity, then the human body movement state determining unit 400 determines that the human body is in a regular fierce movement state.

If the human body movement state determining unit 400 determines that the human body is in the irregular fierce movement state, then accumulates the time periods of the acceleration signals into the total time period of the irregular fierce movement state, accumulates the energy of the acceleration signals into the total energy of the irregular fierce movement state, and the sampling time period setting unit 600 sets the sampling time period of acceleration signals as the sampling time period of the fierce movement state; the storage unit 700 stores the total time period of the irregular fierce movement state and the total energy of the irregular fierce movement state.

If the human body movement state determining unit 400 determines that the human body is in the regular fierce movement state, then accumulates the time periods of the acceleration signals into the total time period of the regular fierce movement state, accumulates the energy of the acceleration signals into the total energy of the regular fierce movement state, and the step counting unit 900 calculates movement step number according to the acceleration signals, and accumulates the movement step number into the total movement step number; the sampling time period setting unit 600 sets the sampling time period of acceleration signals as the sampling time period of fierce movement state; the storage unit 700 stores the total time period of the regular fierce movement state, the total energy of the regular fierce movement state and the movement step number.

Preferably, the human body movement state monitoring device 1000 may further comprise a display unit 950 for optionally displaying the total time period of the sleeping state, the total energy of the sleeping state, the total time period of the sleeping abnormal movements, the total time period of the light movement state, the total energy of the light movement state, the total time period of the irregular fierce movement state, the total energy of the irregular fierce movement state, the total time period of the regular fierce movement state, the total energy of the regular fierce movement state and the total step number of movement.

Hereinbefore the human body movement state monitoring method and device according to the present invention are described by referring to attached drawings in an illustrative way. However, one skilled in the art should understand that, for the human body movement state monitoring method and device of the present invention, various modifications can be made without departing from the content of the present invention. Hence, the protection scope of the present invention should be determined by the content of appended claims.

It should be noted that:
The example of each component in the present invention can be achieved by hardware, or achieved by software module operating on one or more processors, or achieved in a combination thereof. Those skilled in the art should understand that, some or all functions of some or all components according to the example of the present invention can be achieved by using micro-processor or digital signal processor (DSP) in practice. The present invention can also be implemented as a program for carrying out part or all equipment or device in the described method herein (for example, computer program and computer program product). The program implementing the present invention as such can be stored on the computer readable medium, or may be having a form of one or more signals. Such a signal can be downloaded from internet website, or provided on a carrier signal, or provided in any other forms.

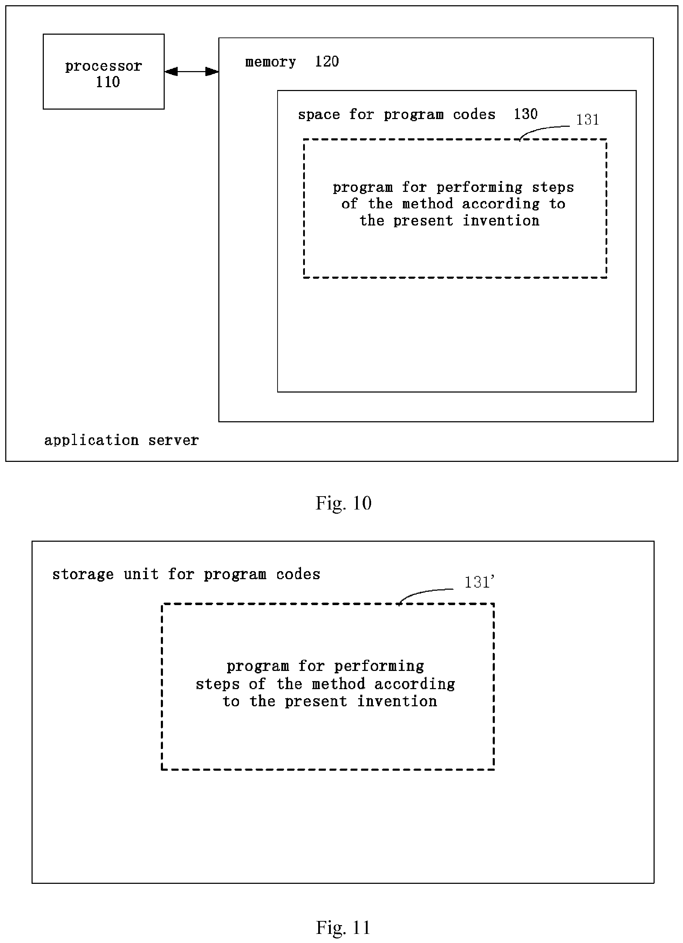

For example, Fig.10 demonstrates a server capable of implementing the human body movement state monitoring method according to the present invention, e.g., an application server. The server conventionally comprises a processor 110 and a computer program product or computer readable medium in a form of memory 120. Memory 120 can be electronic memory, such as flash memory, EEPROM (electrically erasable programmable read only memory), EPROM, hard disk, ROM or the like. Memory 120 has a storage space 130 for the program codes 131 performing the steps of any method step in aforesaid methods. For example, the storage space 130 for program codes can comprise individual program codes 131 respectively for implementing each step in aforesaid method. These program codes can be read out from one or more computer program products or written into one or more computer program products. These computer program products comprise program code carriers such as hard disk, compact disk (CD), memory card or floppy disk and the like. Such computer program products generally are portable or fixed storage unit as shown in Fig.11. The storage unit may have storage segment, storage space and the like set similar to that of the memory 120 in the server of Fig.10. Program codes can be compressed in a suitable form. Generally, the storage unit comprises the computer readable codes 131' for implementing the steps of the method according to the present invention, i.e., the codes can be read from processors such as 110 and the like, when these codes are operated by a server, the server is caused to carry out each step in aforesaid method.

It should be noted that the aforesaid examples are for explaining the present invention rather than limiting the present invention, and those skilled in the art can design alternative example without departing from the scope of appended claims. In the claims, any reference symbol located between parentheses should not be construed as limitation to claims. Word "comprise" does not exclude the existence of element or step not defined in the claims. The present invention can be implemented through hardware containing various different elements and through suitably programmed computer. In the device claim listing several devices, several of these devices can be specifically implemented by one same hardware item.

In the description provided herein, numerous specific details are described. However, it can be understood that, the examples of the present invention can be carried out without these specific details. In some examples, well-known methods, structures and techniques are not disclosed in details, so as not to blur the understanding of the present specification. The terms used in the present specification are mainly selected for the purpose of readability and instruction, rather than selected for explaining or limiting the subject matter of the present invention.

## Claims

1. A human body movement state monitoring method, wherein the method comprises the following steps performed repeatedly:

   a) obtaining acceleration signals having a set sampling time period from output of a triaxial acceleration sensor worn on a human body, and calculating the energy and average power of the acceleration signals;
   b) determining a human body movement state according to the average power of the acceleration signals, and if the average power of the acceleration signals is more than a predetermined fierce movement threshold, determining that the human body is in a fierce movement state, if the average power of the acceleration signals is less than a predetermined sleeping threshold, determining that the human body is in a sleeping state, if the average power of the acceleration signals is less than the fierce movement threshold and is more than the sleeping threshold, determining that the human body is in a light movement state;
   c1) if the human body is in the sleeping state, accumulating time periods of the acceleration signals into a total time period of the sleeping state, accumulating the energy of the acceleration signals into a total energy of the sleeping state, counting up the time periods of acceleration signals which have intensity more than a predetermined intensity threshold, and accumulating the counted time periods into a total time period of sleeping abnormal movements, and setting a sampling time period of acceleration signals as a sampling time period of the sleeping state, then returning to step a);
   c2) if the human body is in the light movement state, accumulating the time periods of the acceleration signals into a total time period of the light movement state, accumulating the energy of the acceleration signals into a total energy of the light movement state, and setting the sampling time period of acceleration signals as a sampling time period of the light movement state, then returning to step a);

c3) if the human body is in the fierce movement state, further determining whether the acceleration signals have quasi-periodicity, if the acceleration signals do not have quasi-periodicity, determining that the human body is in an irregular fierce movement state, if the acceleration signals have quasi-periodicity, determining that the human body is in a regular fierce movement state;

d1) if the human body is in the irregular fierce movement state, accumulating the time periods of the acceleration signals into a total time period of the irregular fierce movement state, accumulating the energy of the acceleration signals into a total energy of the irregular fierce movement state, and setting the sampling time period of acceleration signals as a sampling time period of the fierce movement state, then returning to step a);

d2) if the human body is in the regular fierce movement state, accumulating the time periods of the acceleration signals into a total time period of the regular fierce movement state, accumulating the energy of the acceleration signals into a total energy of the regular fierce movement state, calculating movement step number according to the acceleration signals, and accumulating the movement step number into a total movement step number, and setting the sampling time period of acceleration signals as the sampling time period of the fierce movement state, then returning to step a);

wherein the average power P of the acceleration signals is calculated from the following formula

$$P = \frac{1}{N}\sum_{i=1}^{N}(a_i - a_0)^2 \,,$$

wherein $a_i$ is the No. i value of the acceleration signals, N is the length of the acceleration signals, and $1 \leq i \leq N$, $a_0$ is the average

$$a_0 = \frac{1}{N}\sum_{i=1}^{N}a_i \,.$$

value of the acceleration signals,

2. The human body movement state monitoring method according to claim 1, wherein the determining step of the quasi-periodicity of the acceleration signals comprises:

    performing high-pass filtering on the acceleration signals;
    performing pitch detection on the high-pass filtered acceleration signals;

setting a low-pass or band-pass filter by using the pitch obtained by the pitch detection as cut-off frequency, and using the low-pass or band-pass filter to perform low-pass or band-pass filtering on corresponding high-pass filtered acceleration signals;
obtaining extreme value points of the acceleration signals in the low-pass or band-pass filtered acceleration signals and removing interfering extreme value points in the extreme value points of the acceleration signals, so as to obtain effective extreme value points in the low-pass or band-pass filtered acceleration signals;
calculating time gaps between adjacent effective extreme value points, obtaining a time gap sequence, and calculating differences between adjacent time gaps in the time gap sequence, obtaining a time gap difference sequence, and if each of a continuous predetermined number of time gap differences in the time gap difference sequence is less than a predetermined period threshold, determining that the acceleration signals have quasi-periodicity, otherwise, determining that the acceleration signals do not have quasi-periodicity.

3. The human body movement state monitoring method according to claim 2, wherein the step of calculating movement step number according to the acceleration signals comprises:
counting the effective extreme value points in the low-pass or band-pass filtered acceleration signals having quasi-periodicity, the number of the effective extreme value points being movement step number.

4. The human body movement state monitoring method according to claim 3, further comprises obtaining a displacement signal by double integral of the acceleration signals on time.

5. The human body movement state monitoring method according to claim 2, wherein performing pitch detection on the high-pass filtered acceleration signals comprises:

    attenuating the signals with a filter that attenuates signal energy with an incrementing degree from low frequency to high frequency;
    obtaining the autocorrelation function $\rho(\tau)$ of the attenuated signals from the following formula:

$$\rho(\tau) = \frac{\sum_{n=1}^{N}a(n)a(n-\tau)}{\sqrt{\sum_{n=1}^{N}a^2(n)\sum_{n=0}^{N-1}a^2(n-\tau)}}$$

wherein $a(n)$ is the No. n value of the attenuated signals, N is the length of the signals, and $1 \leq n \leq N$, $\tau$ is a delay time, $\rho(\tau)$ is normalized autocorrelation function of the signals; calculating the value of $\tau$ corresponding to the maximal value of $\rho(\tau)$, and the reciprocal of the $\tau$ value is the pitch of the signals.

6. The human body movement state monitoring method according to claim 2, wherein, removing the interfering extreme value points from the extreme value points of the acceleration signals comprises: filtering out the interfering extreme value points from the extreme value points of the acceleration signals through a time gap; alternatively, filtering out the interfering extreme value points from the extreme value points of the acceleration signals through a time gap and a magnitude value.

7. The human body movement state monitoring method according to claim 6, wherein the interfering extreme value points comprise such an extreme value point of the acceleration signals that the time gap between the extreme value point of the acceleration signals and the previous extreme value point of the acceleration signals is less than a predetermined threshold; or the interfering extreme value points comprise extreme value points of the acceleration signals, whose magnitude values are not maximal, among each group of extreme value points of the acceleration signals with time gaps continuously less than a predetermined threshold.

8. The human body movement state monitoring method according to claim 1, also comprises optionally displaying the total time period of the sleeping state, the total energy of the sleeping state, the total time period of the sleeping abnormal movements, the total time period of the light movement state, the total energy of the light movement state, the total time period of the irregular fierce movement state, the total energy of the irregular fierce movement state, the total time period of the regular fierce movement state, the total energy of the regular fierce movement state and the total step number of movement.

9. A human body movement state monitoring device, wherein the device comprises: a triaxial acceleration sensor (100), an acceleration signal obtaining unit (200), a calculating unit (300), a human body movement state determining unit (400), a sleeping abnormal movement statistical unit (500), a sampling time period setting unit (600), a storage unit (700), a quasi-periodicity determining unit (800) and a step counting unit (900), wherein the acceleration signal obtaining unit (200) obtains acceleration signals having a set sampling time period from output of the triaxial acceleration sensor (100) worn on a human body, and the calculating unit (300) calculates the energy and average power of the acceleration signals;

the human body movement state determining unit (400) determines a human body movement state according to the average power of the acceleration signals, and if the average power of the acceleration signals is more than a predetermined fierce movement threshold, determines that the human body is in a fierce movement state, if the average power of the acceleration signals is less than a predetermined sleeping threshold, determines that the human body is in a sleeping state, if the average power of the acceleration signals is less than the fierce movement threshold and is more than the sleeping threshold, determines that the human body is in a light movement state;

if the human body movement state determining unit (400) determines that the human body is in the sleeping state, accumulates the time periods of the acceleration signals into a total time period of the sleeping state, accumulates the energy of the acceleration signals into a total energy of the sleeping state; the sleeping abnormal movement statistical unit (500) counts up the time periods of acceleration signals which have intensity more than a predetermined intensity threshold, and accumulates the counted time periods into a total time period of sleeping abnormal movements; the sampling time period setting unit (600) sets the sampling time period of acceleration signal as the sampling time period of the sleeping state; the storage unit (700) stores the total time period of the sleeping state, the total energy of the sleeping state and the total time period of the sleeping abnormal movements;

if the human body movement state determining unit (400) determines that the human body is in the light movement state, accumulates the time periods of the acceleration signals into a total time period of the light movement state, accumulates the energy of the acceleration signals into a total energy of the light movement state, and the sampling time period setting unit (600) sets the sampling time period of acceleration signals as the sampling time period of the light movement state; the storage unit (700) stores the total time period of the light movement state and the total energy of light movement state;

if the human body movement state determining unit (400) determines that the human body is in the fierce movement state, then the quasi-period determining unit (800) determines whether the acceleration signals have quasi-periodicity, and if determining that the acceleration signals do not have quasi-periodicity, then the human body movement state determining unit (400) determines that the human body is in an irregular fierce movement state, and if the quasi-period determining unit (800) determines that the acceleration signals have quasi-periodicity, then the human body movement state determining unit (400) determines that the human body is in a regular fierce movement state;

if the human body movement state determining unit (400) determines that the human body is in the irregular fierce movement state, then accumulates the time periods of the acceleration signals into a total time period of the irregular fierce movement state, accumulates the energy of the acceleration signals into a total energy of the irregular fierce movement state, and the sampling time period setting unit (600) sets the sampling time period of acceleration signals as the sampling time period of the fierce movement state; the storage unit (700) stores the total time period of the irregular fierce movement state and the total energy of the irregular fierce movement state;

if the human body movement state determining unit (400) determines that the human body is in the regular fierce movement state, then accumulates the time periods of the acceleration signals into a total time period of the regular fierce movement state, accumulates the energy of the acceleration signals into a total energy of the regular fierce movement state, and the step counting unit (900) calculates movement step number according to the acceleration signals, and accumulates the movement step number into a total movement step number; the sampling time period setting unit (600) sets the sampling time period of acceleration signals as the sampling time period of fierce movement state; the storage unit (700) stores the total time period of the regular fierce movement state, the total energy of the regular fierce movement state and the movement step number;

wherein the average power P of the acceleration signals is calculated from the following formula

$$P = \frac{1}{N} \sum_{i=1}^{N} (a_i - a_0)^2 ,$$

wherein $a_i$ is the No. i value of the acceleration signals, N is the length of the acceleration signals, and $1 \leq i \leq N$, $a_0$ is the average

$$a_0 = \frac{1}{N} \sum_{i=1}^{N} a_i$$

value of the acceleration signals,

10. The human body movement state monitoring device according to claim 9, further comprising a display unit for optionally displaying the total time period of the sleeping state, the total energy of the sleeping state, the total time period of the sleeping abnormal movements, the total time period of the light movement state, the total energy of the light movement state, the total time period of the irregular fierce movement state, the total energy of the irregular fierce movement state, the total time period of the regular fierce movement state, the total energy of the regular fierce movement state and the total step number of movement.

11. A computer program, comprising computer readable codes, wherein when the computer readable codes are operated on a server, the server is caused to carry out the human body movement state monitoring method according to any one of claims 1-8.

12. A computer readable medium that stores the computer program according to claim 11.

**Patentansprüche**

1. Ein Verfahren zum Überwachen des Bewegungszustandes eines menschlichen Körpers, wobei das Verfahren die folgenden Schritte umfasst, die wiederholt durchgeführt werden:

a) Erhalten von Beschleunigungssignalen mit einer festgelegten Abtastzeitperiode aus dem Ausgang eines am menschlichen Körper getragenen triaxialen Beschleunigungssensors und Berechnen der Energie und der mittleren Leistung der Beschleunigungssignale;

b) Bestimmen eines Bewegungszustandes des menschlichen Körpers gemäß der mittleren Leistung der Beschleunigungssignale, und falls die mittlere Leistung der Beschleunigungssignale größer als ein vorbestimmter Schwellenwert für heftige Bewegung ist, Bestimmen, dass der menschliche Körper in einem Zustand heftiger Bewegung ist, falls die mittlere Leistung der Beschleunigungssignale kleiner als ein vorbestimmter Schwellenwert für Schlafen ist, Bestimmen, dass der menschliche Körper in einem Zustand des Schlafens ist, falls die mittlere Leistung der Beschleunigungssignale kleiner als der Schwellenwert für heftige Bewegung und größer als der Schwellenwert für Schlafen ist, Bestimmen, dass der menschliche Körper in einem Zustand leichter Bewegung ist;

c1) falls sich der menschliche Körper im Schlafenzustand befindet, Akkumulieren von Zeitperioden der Beschleunigungssignale in eine Gesamtzeitperiode des Schlafenzustands, Akkumulieren der Energie der Beschleunigungssignale in eine Gesamtenergie des Schlafenzustands, Hochzählen der Zeitperioden der Beschleunigungssignale, welche eine Intensität aufweisen über einem vorgegebenen Intensitätsschwellenwert, und Akkumulieren der gezählten Zeitperioden in eine Gesamtzeitperiode von Schlafen abnormaler Bewegungen, und Festlegen einer Abtastzeitperiode von Beschleunigungssignalen als eine Abtastzeitperiode des Schlafenzustands, dann Rückkehr zu Schritt a);

c2) falls sich der menschliche Körper im Zustand leichter Bewegung befindet, Akkumulieren der Zeitperioden der Beschleunigungssignale in eine Gesamtzeitperiode des Zustandes leichter Bewegung, Akkumulieren der Energie der Beschleunigungssignale in eine Gesamtenergie des Zustandes leichter Bewegung und Einstellen der Abtastzeitperiode der Beschleunigungssignale als eine Abtastzeitperiode des Zustandes leichter Bewegung, dann Rückkehr zu Schritt a);

c3) falls sich der menschliche Körper im Zustand heftiger Bewegung befindet, weiter bestimmen, ob die Beschleunigungssignale eine Quasi-Periodizität aufweisen, falls die Beschleunigungssignale keine Quasi-Periodizität aufweisen, Bestimmen, dass sich der menschliche Körper in einem Zustand irregulär heftiger Bewegung befindet, falls die Beschleunigungssignale eine Quasi-Periodizität aufweisen, Bestimmen, dass sich der menschliche Körper in einem Zustand regulär heftiger Bewegung befindet;

d1) falls sich der menschliche Körper im Zustand irregulär heftiger Bewegung befindet, Akkumulieren der Zeitperioden der Beschleunigungssignale in eine Gesamtzeitperiode des Zustands der irregulär heftigen Bewegung, Akkumulieren der Energie der Beschleunigungssignale in eine Gesamtenergie des Zustands irregulär heftiger Bewegung, und Einstellen der Abtastzeitperiode der Beschleunigungssignale als eine Abtastzeitperiode des Zustands heftiger Bewegung, dann Rückkehr zu Schritt a);

d2) falls sich der menschliche Körper im Zustand regulär heftiger Bewegung befindet, Akkumulieren der Zeitperioden der Beschleunigungssignale in eine Gesamtzeitperiode des Zustands regulär heftiger Bewegung, Akkumulieren der Energie der Beschleunigungssignale in eine Gesamtenergie des Zustands regulär heftiger Bewegung, Berechnen der Bewegungsschrittzahl gemäß den Beschleunigungssignalen und Akkumulieren der Bewegungsschrittzahl in eine Gesamtbewegungsschrittzahl und Einstellen der Abtastzeitperiode der Beschleunigungssignale als die Abtastzeitperiode des Zustands heftiger Bewegung, dann Rückkehr zu Schritt a);

wobei die mittlere Leistung P der Beschleunigungssignale aus der folgenden Formel

$$P = \frac{1}{N} \sum_{i=1}^{N} (a_i - a_0)^2$$

berechnet wird, wobei *ai* der Nr. i Wert der Beschleunigungssignale ist, N die Länge der Beschleunigungssignale ist und $1 \leq i \leq N$, *a0* der Mittelwert der Beschleunigungssignale ist,

$$a_0 = \frac{1}{N} \sum_{i=1}^{N} a_i .$$

2. Das Verfahren zum Überwachen des Bewegungszustandes eines menschlichen Körpers nach Anspruch 1, wobei der Bestimmungsschritt der Quasi-Periodizität der Beschleunigungssignale aufweist:

Durchführen einer Hochpassfilterung der Beschleunigungssignale;
Durchführen einer Pitch-Erkennung an den hochpassgefilterten Beschleunigungssignalen;
Einstellen eines Tiefpaß- oder Bandpassfilters unter Verwendung des durch die Pitch-Erkennung erhaltenen Pitches als Grenzfrequenz und Verwenden des Tiefpaß- oder Bandpassfilters zum Durchführen einer Tiefpaß- oder Bandpassfilterung auf entsprechenden hochpaßgefilterten Beschleunigungssignalen;
Erhalten von Extremwertpunkten der Beschleunigungssignale in den tiefpaß- oder bandpaßgefilterten Beschleunigungssignalen und Entfernen störender Extremwertpunkte in den Extremwertpunkten der Beschleunigungssignale, um so effektive Extremwertpunkte in den tiefpaß- oder bandpaßgefilterten Beschleunigungssignalen zu erhalten;
Berechnen von Zeitabständen zwischen benachbarten effektiven Extremwertpunkten, Erhalten einer Zeitabstandsfolge, und Berechnen von Differenzen zwischen benachbarter Zeitabstände in der Zeitabstandsfolge, Erhalten einer Zeitabstandsdifferenzfolge, und falls jede einer kontinuierlichen vorbestimmten Anzahl von Zeitabstandsdifferenzen in der Zeitabstandsdifferenzfolge kleiner als ein vorbestimmter Periodenschwellenwert ist, Bestimmen, dass die Beschleunigungssignale eine Quasi-Periodizität aufweisen, andernfalls Bestimmen, dass die Beschleunigungssignale keine Quasi-Periodizität aufweisen.

3. Das Verfahren zum Überwachen des Bewegungszustands des menschlichen Körpers nach Anspruch 2, wobei der Schritt des Berechnens der Bewegungsschrittzahl gemäß den Beschleunigungssignalen aufweist:
Zählen der effektiven Extremwertpunkte in den tiefpaß- oder bandpaßgefilterten Beschleunigungssignalen mit Quasi-Periodizität, wobei die Zahl der effektiven Extremwertpunkte Bewegungsschrittzahl ist.

4. Das Verfahren zum Überwachen des Bewegungszustands des menschlichen Körpers nach Anspruch 3 umfasst ferner das Erhalten eines Versatzsignals durch doppeltes Integral der Beschleunigungssignale über Zeit.

5. Das Verfahren zum Überwachen des Bewegungszustands des menschlichen Körpers nach Anspruch 2, wobei das Durchführen der Pitch-Erkennung an den hochpaßgefilterten Beschleunigungssignalen aufweist:

    Dämpfen der Signale mit einem Filter, der die Signalenergie mit einem von niedriger Frequenz zu hoher Frequenz inkrementierenden Grad dämpft;
    Erhalten der Autokorrelationsfunktion $\rho(\tau)$ der gedämpften Signale aus der folgenden Formel:

$$\rho(\tau) = \frac{\sum_{n=1}^{N} a(n)a(n-\tau)}{\sqrt{\sum_{n=1}^{N} a^2(n) \sum_{n=0}^{N-1} a^2(n-\tau)}}$$

    wobei $a(n)$ der Nr. n Wert der gedämpften Signale ist, N die Länge der Signale ist und $1 \leq n \leq N$, $\tau$ eine Verzögerungszeit ist, $\rho(\tau)$ normalisierte Autokorrelationsfunktion der Signale ist;
    Berechnen des Wertes von $\tau$ entsprechend dem Maximalwert von $\rho(\tau)$, und der Kehrwert des $\tau$ Wertes ist der Pitch der Signale.

6. Das Verfahren zum Überwachen des Bewegungszustandes des menschlichen Körpers nach Anspruch 2, wobei das Entfernen der störenden Extremwertpunkte von den Extremwertpunkten der Beschleunigungssignale aufweist: Herausfiltern der störenden Extremwertpunkte aus den Extremwertpunkten der Beschleunigungssignale über einen Zeitabstand; alternativ Herausfiltern der störenden Extremwertpunkte aus den Extremwertpunkten der Beschleunigungssignale über einen Zeitabstand und einen Größenwert.

7. Das Verfahren zum Überwachen des Bewegungszustandes des menschlichen Körpers nach Anspruch 6, wobei die störenden Extremwertpunkte einen solchen Extremwertpunkt der Beschleunigungssignale aufweist, dass der Zeitabstand zwischen dem Extremwertpunkt der Beschleunigungssignale und dem vorhergehenden Extremwertpunkt der Beschleunigungssignale kleiner als ein vorbestimmter Schwellenwert ist; oder die störenden Extremwertpunkte Extremwertpunkte der Beschleunigungssignale aufweisen, deren Größenwerte nicht maximal sind, zwischen jeder Gruppe von Extremwertpunkten der Beschleunigungssignale mit Zeitabständen, die kontinuierlich kleiner als ein vorbestimmter Schwellenwert sind.

8. Das Verfahren zum Überwachen des Bewegungszustandes des menschlichen Körpers nach Anspruch 1 weist auch das optionale Anzeigen der Gesamtzeitperiode des Schlafenzustandes, der Gesamtenergie des Schlafenzustandes, der Gesamtzeitperiode der Schlafen abnormalen Bewegung, der Gesamtzeitperiode des Zustands leichter Bewegung, der Gesamtenergie des Zustands leichter Bewegung, der Gesamtzeitperiode des Zustands irregulär heftiger Bewegung, der Gesamtenergie des Zustandes irregulär heftiger Bewegung, der Gesamtzeitperiode des Zustands regulär heftiger Bewegung, der Gesamtenergie des Zustandes regulär heftiger Bewegung und der Gesamtschrittzahl der Bewegung auf.

9. Eine Vorrichtung zur Überwachung des Bewegungszustands des menschlichen Körpers, wobei die Vorrichtung aufweist:

    einen triaxialen Beschleunigungssensor (100), eine Einheit (200) zum Erhalten eines Beschleunigungssignals, eine Berechneneinheit (300), eine Einheit (400) zum Bestimmen des Bewegungszustands des menschlichen Körpers, eine Schlafen abnormal Bewegung-statistische Einheit (500), eine Abtastzeitperiode-Einstellen-Einheit (600), eine Speichereinheit (700), eine Quasi-Periodizität-Bestimmen-Einheit (800) und eine Schrittzähleinheit (900), wobei
    die Einheit (200) zum Erhalten eines Beschleunigungssignals Beschleunigungssignale mit einer eingestellten Abtastzeitperiode von dem Ausgang des an einem menschlichen Körper getragenen triaxialen Beschleunigungssensors (100) erhält, und die Berechneneinheit (300) die Energie und die mittlere Leistung der Beschleunigungssignale berechnet;
    die Einheit (400) zum Bestimmen des Bewegungszustands des menschlichen Körpers einen Bewegungszustand des menschlichen Körpers gemäß der mittleren Leistung der Beschleunigungssignale bestimmt, und falls die mittlere Leistung der Beschleunigungssignale größer als ein vorbestimmter Schwellenwert für heftige Bewegung ist, bestimmt, dass sich der menschliche Körper in einem Zustand heftiger Bewegung befindet, falls die mittlere Leistung der Beschleunigungssignale kleiner als ein vorbestimmter Schlafen-Schwellenwert ist, bestimmt, dass sich der menschliche Körper in einem Schlafenzustand befindet, falls die mittlere Leistung der Beschleunigungssignale kleiner als der Schwellenwert für heftige Bewegung ist und größer als der Schlafen-Schwellenwert ist, bestimmt, dass sich der menschliche Körper in einem Zustand leichter Bewegung befindet;
    falls die Einheit (400) zum Bestimmen des Bewegungszustands des menschlichen Körpers bestimmt, dass sich der menschliche Körper im Schlafenzustand befindet, die Zeitperioden der Beschleunigungssignale in eine Gesamtzeitperiode des Schlafenzustands akkumuliert, die Energie der Beschleunigungssignale in eine Gesamtenergie des Schlafzustands akkumuliert; die Schlafen abnormal Bewegung-statistische Einheit (500) die Zeitperioden der Beschleunigungssignale hochzählt, die eine Intensität von mehr als einem vorbestimmten Intensitätsschwellenwert aufweisen, und die gezählten Zeitperioden in eine Gesamtzeitperiode von Schlafen-abnormaler Bewegungen akkumuliert; die Abtastzeitperioden-Einstellen-Einheit (600) die Abtastzeitperiode des Beschleunigungssignals als die Abtastzeitperiode des Schlafenzustands einstellt; die Speichereinheit (700) die Gesamtzeitperiode des Schlafenzustands, die Gesamtenergie des Schlafenzustands und die Gesamtzeitperiode der Schlafen-abnormalen Bewegungen speichert;

falls die Einheit (400) zum Bestimmen des Bewegungszustands des menschlichen Körpers bestimmt, dass sich der menschliche Körper im Zustand leichter Bewegung befindet, die Zeitperioden der Beschleunigungssignale in eine Gesamtzeitperiode des Zustands leichter Bewegung akkumuliert, die Energie der Beschleunigungssignale in eine Gesamtenergie des Zustands leichter Bewegung akkumuliert, und die Abtastzeitperioden-Einstellen-Einheit (600) die Abtastzeitperiode der Beschleunigungssignale als die Abtastzeitperiode des Zustands leichter Bewegung einstellt; die Speichereinheit (700) die Gesamtzeitperiode des Zustands leichter Bewegung und die Gesamtenergie des Zustands leichter Bewegung speichert;

falls die Einheit (400) zum Bestimmen des Bewegungszustands des menschlichen Körpers bestimmt, dass sich der menschliche Körper in dem Zustand heftiger Bewegung befindet, dann bestimmt die Quasi-Periode-Bestimmen-Einheit (800), ob die Beschleunigungssignale eine Quasi-Periodizität haben, und falls bestimmt wird, dass die Beschleunigungssignale keine Quasi-Periodizität haben, dann bestimmt die Einheit (400) zum Bestimmen des Bewegungszustands des menschlichen Körpers, dass sich der menschliche Körper in einem Zustand irregulär heftiger Bewegung befindet, und falls die Quasi-Periode-Bestimmen-Einheit (800) bestimmt, dass die Beschleunigungssignale eine Quasi-Periodizität aufweisen, dann bestimmt die den Einheit (400) zum Bestimmen des Bewegungszustands des menschlichen Körpers, dass sich der menschliche Körper in einem Zustand regulär heftiger Bewegung befindet;

falls die Einheit (400) zum Bestimmen des Bewegungszustands des menschlichen Körpers bestimmt, dass sich der menschliche Körper in dem Zustand irregulär heftiger Bewegung befindet, dann die Zeitperioden der Beschleunigungssignale in eine Gesamtzeitperiode des Zustands irregulär heftiger Bewegung akkumuliert, die Energie der Beschleunigungssignale in eine einer Gesamtenergie des Zustands irregulär heftiger Bewegung akkumuliert, und die Abtastzeitperioden-Einstellen-Einheit (600) die Abtastzeitperiode der Beschleunigungssignale als die Abtastzeitperiode des Zustands heftiger Bewegung einstellt; die Speichereinheit (700) die Gesamtzeitperiode des Zustands irregulär heftiger Bewegung und die Gesamtenergie des Zustands irregulär heftiger Bewegung speichert;

falls die Einheit (400) zum Bestimmen des Bewegungszustands des menschlichen Körpers bestimmt, dass sich der menschliche Körper in einem Zustand regulär heftiger Bewegung befindet, dann die Zeitperioden der Beschleunigungssignale in eine Gesamtzeitperiode des Zustands regulär heftiger Bewegung akkumuliert, die Energie der Beschleunigungssignale in eine Gesamtenergie des Zustands regulär heftiger Bewegung akkumuliert, und die Schrittzähleinheit (900) die Bewegungsschrittzahl gemäß den Beschleunigungssignalen berechnet und die Bewegungsschrittzahl in eine Gesamtbewegungsschrittzahl akkumuliert; die Abtastzeitperioden-Einstell-Einheit (600) die Abtastzeitperiode der Beschleunigungssignale als die Abtastzeitperiode des Zustands heftiger Bewegung einstellt; die Speichereinheit (700) die Gesamtzeitperiode des Zustands regulär heftiger Bewegung, die Gesamtenergie des Zustands regulär heftiger Bewegung und die Bewegungsschrittzahl speichert;

wobei die mittlere Leistung P der Beschleunigungssignale aus der folgenden Formel

$$P = \frac{1}{N} \sum_{i=1}^{N} (a_i - a_0)^2$$

berechnet wird, wobei $a_i$ der Nr. i Wert der Beschleunigungssignale ist, N die Länge der Beschleunigungssignale ist und $1 \leq i \leq N$, $a_0$ der Mittelwert der Beschleunigungssignale ist,

$$a_0 = \frac{1}{N} \sum_{i=1}^{N} a_i$$

.

10. Die Vorrichtung zur Überwachung des Bewegungszustands des menschlichen Körpers nach Anspruch 9, ferner aufweisend eine Anzeigeeinheit zum optionalen Anzeigen der Gesamtzeitperiode des Schlafenzustands, der Gesamtenergie des Schlafenzustands, der Gesamtzeitperiode der Schlafen-abnormalen Bewegungen, der Gesamtzeitperiode des Zustands leichter Bewegung, der Gesamtenergie des Zustands leichter Bewegung, der Gesamtzeitperiode des Zustands irregulär heftiger Bewegung, der Gesamtenergie des Zustands irregulär heftiger Bewegung, der Gesamtzeitperiode des Zustands regulär heftiger Bewegung, der Gesamtenergie des Zustands regulär heftiger Bewegung und der Gesamtschrittzahl der Bewegung.

11. Ein Computerprogramm, das computerlesbare Codes aufweist, wobei wenn die computerlesbaren Codes auf einem Server betrieben werden, der Server veranlasst wird, das Verfahren zur Überwachung des Bewegungszustands des menschlichen Körpers gemäß einem der Ansprüche 1-8 auszuführen.

12. Ein computerlesbares Medium, das das Computerprogramm nach Anspruch 11 speichert.

**Revendications**

1. Une méthode de surveillance de l'état des mouvements du corps humain, dans laquelle la méthode comprend les étapes suivantes réalisées de manière répétée :

a) obtenir des signaux d'accélération ayant une période d'échantillonnage déterminée à partir de la sortie d'un capteur d'accélération triaxial porté sur un corps humain, et calculer l'énergie et la puissance moyenne des signaux d'accélération ;

b) déterminer un état de mouvement du corps humain en fonction de la puissance moyenne des signaux d'accélération, et si la puissance moyenne des signaux d'accélération est supérieure à un seuil de mouvement féroce prédéterminé, déterminer que le corps humain est dans un état de mouvement féroce, si la puissance moyenne des signaux d'accélération est inférieure à un seuil de sommeil prédéterminé, déterminer que le corps humain est dans un état de sommeil, si la puissance moyenne des signaux d'accélération est inférieure au seuil de mouvement féroce et est supérieure au seuil de sommeil, déterminer que le corps humain est dans un état de mouvement léger ;

c1) si le corps humain est en état de sommeil, accumuler les périodes de temps des signaux d'accélération en une période de temps totale de l'état de sommeil, accumuler l'énergie des signaux d'accélération en une énergie totale de l'état de sommeil, compter les périodes de temps des signaux d'accélération qui ont une intensité supérieure à un seuil d'intensité prédéterminé, et accumuler les périodes de temps comptées en une période de temps totale de mouvements anormaux de sommeil, et définir une période de temps d'échantillonnage des signaux d'accélération comme période de temps d'échantillonnage de l'état de sommeil, puis revenir à l'étape a) ;

c2) si le corps humain est en état de mouvement léger, accumuler les périodes de temps des signaux d'accélération en une période de temps totale de l'état de mouvement léger, accumuler l'énergie des signaux d'accélération en une énergie totale de l'état de mouvement léger, et définir la période de temps d'échantillonnage des signaux d'accélération comme une période de temps d'échantillonnage de l'état de mouvement léger, puis revenir à l'étape a);

c3) si le corps humain est dans un état de mouvement féroce, déterminer en outre si les signaux d'accélération ont une quasi-périodicité, si les signaux d'accélération n'ont pas de quasi-périodicité, déterminer que le corps humain est dans un état de mouvement féroce irrégulier, si les signaux d'accélération ont une quasi-périodicité, déterminer que le corps humain est dans un état de mouvement féroce régulier;

d1) si le corps humain est dans un état de mouvement irrégulier et féroce, accumuler les périodes de temps des signaux d'accélération en une période de temps totale de l'état de mouvement irrégulier et féroce, accumuler l'énergie des signaux d'accélération en une énergie totale de l'état de mouvement irrégulier et féroce, et définir la période de temps d'échantillonnage des signaux d'accélération comme une période de temps d'échantillonnage de l'état de mouvement féroce, puis revenir à l'étape a);

d2) si le corps humain est dans l'état de mouvement féroce régulier, accumuler les périodes de temps des signaux d'accélération en une période de temps totale de l'état de mouvement féroce régulier, accumuler l'énergie des signaux d'accélération en une énergie totale de l'état de mouvement féroce régulier, calculer le nombre de pas de mouvement en fonction des signaux d'accélération, et accumuler le nombre de pas de mouvement en un nombre de pas de mouvement total, et définir la période de temps d'échantillonnage des signaux d'accélération comme la période de temps d'échantillonnage de l'état de mouvement féroce, puis revenir à l'étape a);

dans laquelle la puissance moyenne P des signaux d'accélération est calculée à partir de la formule sui-

$$P = \frac{1}{N}\sum_{i=1}^{N}(a_i - a_0)^2,$$

vante dans laquelle $a_i$ est la valeur n° i des signaux d'accélération, N est la longueur des signaux d'accélération, et $1 \le i \le N$, $a_0$ est la valeur moyenne des signaux d'accélération,

$$a_0 = \frac{1}{N}\sum_{i=1}^{N} a_i.$$

2. Méthode de surveillance de l'état des mouvements du corps humain selon la revendication 1, dans laquelle l'étape de détermination de la quasi-périodicité des signaux d'accélération comprend:

> effectuer un filtrage passe-haut sur les signaux d'accélération ;
> effectuer une détection de tangage sur les signaux d'accélération filtrés passe-haut ;
> définir d'un filtre passe-bas ou passe-bande en utilisant le pas obtenu par la détection du pas comme fréquence de coupure, utiliser du filtre passe-bas ou passe-bande pour effectuer un filtrage passe-bas ou passe-bande sur les signaux d'accélération filtrés passe-haut correspondants;
> obtenir les points de valeur extrême des signaux d'accélération dans les signaux d'accélération filtrés passe-bas ou passe-bande et supprimer les points de valeur extrême interférents dans les points de valeur extrême des signaux d'accélération, de manière à obtenir des points de valeur extrême effectifs dans les signaux d'accélération filtrés passe-bas ou passe-bande;
> calculer les écarts de temps entre des points de valeur extrême effective adjacents, obtenir une séquence d'écarts de temps, et calculer les différences entre des écarts de temps adjacents dans la séquence d'écarts de temps, obtenir une séquence de différence d'écarts de temps, et si chacun d'un nombre prédéterminé continu de différences d'écarts de temps dans la séquence de différence d'écarts de temps est inférieur à un seuil de période prédéterminé, déterminer que les signaux d'accélération ont une quasi-périodicité, sinon, déterminer que les signaux d'accélération n'ont pas de quasi-périodicité.

3. Méthode de surveillance de l'état des mouvements du corps humain selon la revendication 2, dans laquelle l'étape de calcul du numéro d'étape du mouvement en fonction des signaux d'accélération comprend:
compter les points de valeur extrême effective dans les signaux d'accélération filtrés passe-bas ou passe-bande ayant une quasi-périodicité, le nombre de points de valeur extrême effective étant le numéro de pas de mouvement.

4. Méthode de surveillance de l'état des mouvements du corps humain selon la revendication 3, comprend en outre obtenir d'un signal de déplacement par double intégrale des signaux d'accélération dans le temps.

5. Méthode de surveillance de l'état des mouvements du corps humain selon la revendication 2, dans laquelle la détection du tangage sur les signaux d'accélération filtrés passe-haut comprend :

> atténuer des signaux avec un filtre qui atténue l'énergie du signal avec un degré d'incrémentation de la basse fréquence à la haute fréquence ;
> obtenir la fonction d'autocorrélation des $\rho(\tau)$ signaux atténués à partir de la formule suivante:

$$\rho(\tau) = \frac{\sum_{n=1}^{N} a(n)a(n-\tau)}{\sqrt{\sum_{n=1}^{N} a^2(n)\sum_{n=0}^{N-1} a^2(n-\tau)}}$$

> où est la $a(n)$ valeur n des signaux atténués, N est la longueur des signaux, et $1 \le n \le N$, est $\tau$ un temps de retard, est une fonction d'autocorrélation $\rho(\tau)$ normalisée des signaux;
> calculer la valeur de correspondant à la r valeur maximale de $\rho(\tau)$, et la réciproque de la r valeur est le pas des signaux.

6. Méthode de surveillance de l'état des mouvements du corps humain selon la revendication 2, dans lequel éliminer des points de valeurs extrêmes interférents des points de valeurs extrêmes des signaux d'accélération comprend: filtrer des points de valeurs extrêmes interférents des points de valeurs extrêmes des signaux d'accélération à travers un intervalle de temps; ou bien, filtrer des points de valeurs extrêmes interférents des points de valeurs extrêmes des signaux d'accélération à travers un intervalle de temps et une valeur d'amplitude.

7. Méthode de surveillance de l'état des mouvements du corps humain selon la revendication 6, dans lequel les points de valeurs extrêmes perturbateurs comprennent un point de valeurs extrêmes des signaux d'accélération tel que l'intervalle de temps entre le point de valeurs extrêmes des signaux d'accélération et le point de valeurs extrêmes précédent des signaux d'accélération est inférieur à un seuil prédéterminé; ou les points de valeurs extrêmes perturbateurs comprennent des points de valeurs extrêmes des signaux d'accélération, dont les valeurs de grandeur ne sont pas maximales, parmi chaque groupe de points de valeurs extrêmes des signaux d'accélération avec des intervalles de temps continuellement inférieurs à un seuil prédéterminé.

8. Méthode de surveillance de l'état des mouvements du corps humain selon la revendication 1, comprend également afficher optionnel de la période de temps totale de l'état de sommeil, de l'énergie totale de l'état de sommeil, de la période de temps totale des mouvements anormaux de sommeil, de la période de temps totale de l'état de mouvement léger, de l'énergie totale de l'état de mouvement léger, de la période de temps totale de l'état de mouvement féroce irrégulier, de l'énergie totale de l'état de mouvement féroce irrégulier, de la période de temps totale de l'état de mouvement féroce régulier, de l'énergie totale de l'état de mouvement féroce régulier et du nombre total de pas du mouvement.

9. Un dispositif de surveillance de l'état des mouvements du corps humain, dans laquelle le dispositif comprend: un capteur d'accélération triaxial (100), une unité d'obtention de signal d'accélération (200), une unité de calcul (300), une unité de détermination de l'état de mouvement du corps humain (400), une unité statistique de mouvement anormal en sommeil (500), une unité de réglage de période d'échantillonnage (600), une unité de stockage (700), une unité de détermination de quasi-périodicité (800) et une unité de comptage de pas (900), dans lequel

l'unité d'obtention de signal d'accélération (200) obtient des signaux d'accélération ayant une période d'échantillonnage définie à partir de la sortie du capteur d'accélération triaxial (100) porté sur un corps humain, et l'unité de calcul (300) calcule l'énergie et la puissance moyenne des signaux d'accélération;

l'unité de détermination de l'état de mouvement du corps humain (400) détermine un état de mouvement du corps humain selon la puissance moyenne des signaux d'accélération, et si la puissance moyenne des signaux d'accélération est supérieure à un seuil de mouvement féroce prédéterminé, détermine que le corps humain est dans un état de mouvement féroce, si la puissance moyenne des signaux d'accélération est inférieure à un seuil de sommeil prédéterminé, détermine que le corps humain est dans un état de sommeil, si la puissance moyenne des signaux d'accélération est inférieure au seuil de mouvement féroce et est supérieure au seuil de sommeil, détermine que le corps humain est dans un état de mouvement léger;

si l'unité de détermination des états de mouvement du corps humain (400) détermine que le corps humain est en état de sommeil, accumule les périodes de temps des signaux d'accélération en une période de temps totale de l'état de sommeil, accumule l'énergie des signaux d'accélération en une énergie totale de l'état de sommeil; l'unité statistique des mouvements anormaux du sommeil (500) compte les périodes de temps des signaux d'accélération qui ont une intensité supérieure à un seuil d'intensité prédéterminé, et accumule les périodes de temps comptées en une période de temps totale de mouvements anormaux du sommeil; l'unité de réglage de la période d'échantillonnage (600) définit la période d'échantillonnage du signal d'accélération comme étant la période d'échantillonnage de l'état de sommeil; l'unité de stockage (700) stocke la période totale de l'état de sommeil, l'énergie totale de l'état de sommeil et la période totale des mouvements anormaux du sommeil ;

si l'unité de détermination de l'état de mouvement du corps humain (400) détermine que le corps humain est dans l'état de mouvement léger, accumule les périodes de temps des signaux d'accélération en une période de temps totale de l'état de mouvement léger, accumule l'énergie des signaux d'accélération en une énergie totale de l'état de mouvement léger, et l'unité de réglage de la période de temps d'échantillonnage (600) règle la période de temps d'échantillonnage des signaux d'accélération comme étant la période de temps d'échantillonnage de l'état de mouvement léger; l'unité de stockage (700) stocke la période de temps totale de l'état de mouvement léger et l'énergie totale de l'état de mouvement léger;

si l'unité de détermination de l'état de mouvement du corps humain (400) détermine que le corps humain est dans un état de mouvement féroce, alors l'unité de détermination de la quasi-période (800) détermine si les signaux d'accélération ont une quasi-périodicité, et si elle détermine que les signaux d'accélération n'ont pas de quasi-périodicité, alors l'unité de détermination de l'état de mouvement du corps humain (400) détermine que le corps humain est dans un état de mouvement féroce irrégulier, et si l'unité de détermination de la quasi-période (800) détermine que les signaux d'accélération ont une quasi-périodicité, alors l'unité de détermination de l'état de mouvement du corps humain (400) détermine que le corps humain est dans un état de mouvement féroce régulier;

si l'unité de détermination de l'état de mouvement du corps humain (400) détermine que le corps humain est dans un état de mouvement irrégulier et féroce, alors elle accumule les périodes de temps des signaux d'accélération en une période de temps totale de l'état de mouvement irrégulier et féroce, accumule l'énergie des signaux d'accélération en une énergie totale de l'état de mouvement irrégulier et féroce, et l'unité de réglage de la période de temps d'échantillonnage (600) définit la période de temps d'échantillonnage des signaux d'accélération comme étant la période de temps d'échantillonnage de l'état de mouvement féroce; l'unité de stockage (700) stocke la période de temps totale de l'état de mouvement féroce irrégulier et l'énergie totale de l'état de mouvement féroce irrégulier;

si l'unité de détermination de l'état de mouvement du corps humain (400) détermine que le corps humain est dans l'état de mouvement féroce régulier, alors elle accumule les périodes de temps des signaux d'accélération en une période de temps totale de l'état de mouvement féroce régulier, accumule l'énergie des signaux d'accélération en une énergie totale de l'état de mouvement féroce régulier, et l'unité de comptage des pas (900) calcule le nombre de pas de mouvement en fonction des signaux d'accélération, et accumule le nombre de pas de mouvement en un nombre total de pas de mouvement; l'unité de réglage de la période d'échantillonnage (600) définit la période d'échantillonnage des signaux d'accélération comme la période d'échantillonnage de l'état de mouvement rapide; l'unité de stockage (700) stocke la période totale de l'état de mouvement rapide régulier, l'énergie totale de l'état de mouvement rapide régulier et le numéro de pas de mouvement;

dans laquelle la puissance moyenne P des signaux d'accélération est calculée à partir de la formule suivante

$$P = \frac{1}{N} \sum_{i=1}^{N} (a_i - a_0)^2 ,$$

dans laquelle $a_i$ est la valeur n° i des signaux d'accélération, N est la longueur des signaux d'accélération, et $1 \leq i \leq N$, $a_0$ est la valeur moyenne des signaux d'accélération,

$$a_0 = \frac{1}{N} \sum_{i=1}^{N} a_i .$$

10. Dispositif de surveillance de l'état des mouvements du corps humain selon la revendication 9, comprenant en outre une unité d'affichage pour afficher facultativement la période de temps totale de l'état de sommeil, l'énergie totale de l'état de sommeil, la période de temps totale des mouvements anormaux de sommeil, la période de temps totale de l'état de mouvement léger, l'énergie totale de l'état de mouvement léger, la période de temps totale de l'état de mouvement féroce irrégulier, l'énergie totale de l'état de mouvement féroce irrégulier, la période de temps totale de l'état de mouvement féroce régulier, l'énergie totale de l'état de mouvement féroce régulier et le nombre total de pas du mouvement.

11. Un programme informatique, comprenant des codes lisibles par ordinateur, dans laquelle lorsque les codes lisibles par ordinateur sont exploités sur un serveur, le serveur est amené à exécuter la méthode de surveillance de l'état des mouvements du corps humain selon l'une des revendications 1-8.

12. Un support lisible par ordinateur qui stocke le programme informatique selon la revendication 11.

Fig. 1

Fig. 2

Fig. 3

Fig. 4

S10 — obtaining acceleration signals

S20 — average power is more than fierce movement threshold?

yes → S30 fierce movement state

no

S40 — average power is less than sleeping threshold?

S70 — acceleration signals have quasi-periodicity?

no → S80 irregular fierce movement

yes → S90 regular fierce movement

S50 yes → sleeping state

S60 no → light movement state

S80 / S85:
accumulating time periods of signals,

accumulating energy of signals,

setting sampling time period as the sampling time period of fierce movement state

S90 / S95:
accumulating time periods of signals,

accumulating energy of signals,

calculating movement step number,

setting sampling time period as the sampling time period of fierce movement state

S50 / S55:
accumulating time periods of signals,

accumulating energy of signals,

accumulating time periods of acceleration signals which have intensity more than predetermined threshold,

setting sampling time period as sampling time period of sleeping state

S60 / S65:
accumulating time periods of signals,

accumulating energy of signals,

setting sampling time period as the sampling time period of slight movement state

Fig. 5

Fig. 6a

Fig. 6b

Fig. 6c

Fig. 6d

Fig. 7

Fig. 8

human body movement state monitoring device 1000

Fig. 9

processor
110

memory    120

space for program codes    130

131

program for performing steps
of the method according to
the present invention

application server

Fig. 10

storage unit for program codes

131'

program for performing
steps of the method according
to the present invention

Fig. 11

**EP 2 962 637 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0700661 A2